# NEW EUROPEAN PATENT SPECIFICATION

(11) **EP 3 069 620 B2**
(45) Date of publication and mention of the opposition decision: **24.02.2021**
(45) Mention of the grant of the patent: 18.10.2017
(21) Application number: 15159775.4
(22) Date of filing: 19.03.2015
(51) Int. Cl.: A24F 47/00

(54) **ELECTRONIC SMOKING DEVICE**
ELEKTRONISCHE RAUCHVORRICHTUNG
DISPOSITIF À FUMER ÉLECTRONIQUE

(43) Date of publication of application: 21.09.2016
(73) Proprietor: Fontem Holdings 1 B.V., 1083 HN Amsterdam (NL)
(72) Inventor: Borkovec, Vaclav, 22761 Hamburg (DE)
(74) Representative: Gulde & Partner

(56) References cited:
- EP-A2- 0 430 559
- WO-A1-2011/063970
- WO-A1-2014/160055
- WO-A1-2016/099276
- US-A1- 2014 314 397
- US-A1- 2015 059 787
- Twisted Messes: "Staged heating coil", YouTube video, 10 December 2014 (2014-12-10),
- "Staged heating coil", Reddit, 18 February 2015 (2015-02-18),
- Luxor Vapes' tweet of October 23, 2014 (https://twitter.com/LuxorVapes/status/525 398913587884033)
- Luxor Vapes' tweet of December 3, 2014 (https://twitter.com/LuxorVapes/status/540 275641031991296)
- Cincy Vapors' tweet of August, 19, 2014 (https://twitter.com/CincyVapors/status/50 1769602842382337)
- Luxor Vapes' tweet of October 11, 2014 (https://twitter.com/LuxorVapes/status/521 122788468604928)
- Luxor Vapes' tweet of December 13, 2014 (https://twitter.com/LuxorVapes/status/543 881807263842304)

## Description

### FIELD OF INVENTION

The present invention relates generally to electronic smoking devices and in particular electronic cigarettes.

### BACKGROUND OF THE INVENTION

An electronic smoking device, such as an electronic cigarette (e-cigarette), typically has a housing accommodating an electric power source (e.g. a single use or rechargeable battery, electrical plug, or other power source), and an electrically operable atomizer. The atomizer vaporizes or atomizes liquid supplied from a reservoir and provides vaporized or atomized liquid as an aerosol. Control electronics control the activation of the atomizer. In some electronic cigarettes, an airflow sensor is provided within the electronic smoking device which detects a user puffing on the device (e.g., by sensing an under-pressure or an air flow pattern through the device). The airflow sensor indicates or signals the puff to the control electronics to power up the device and generate vapor. In other e-cigarettes, a switch is used to power up the e-cigarette to generate a puff of vapour.

An electronic smoking devise of the prior art is disclosed in EP 0 430 559 A2. In the YouTube video "Staged heating coil" published on December 10, 2014, retrieved from https://www.youtube.com/watch?v=CzxaTKO_Clo a heating coil is disclosed. Further state of the art is disclosed in the Reddit post "Staged heating coil" published on February 18, 2015, retrieved from https://www.reddit.com/r/Coilporn/comments/2waoch/staged_heating_coil/. The WO 2016/099276 A1 discloses an electrically heated smoking system and the WO 2011/063970 A1 discloses a wick having at least one string.

### SUMMARY OF THE INVENTION

In one aspect an atomizer for an electronic smoking device is provided which comprises at least a first heating wire and a second heating wire. The first and second heating wires are wound together to form a common heating coil. Further, the first and second heating wires differ in at least one physical parameter resulting in different thermal properties of the first and second heating wire. The diameter of a turn of the first heating wire differs from a diameter of a turn of the second heating wire, and the inner winding diameter of the first heating wire differs from the inner winding diameter of the second heating wire.

Different physical parameters of the first and second heating wires may relate to at least one of the following non exclusive list of physical parameters of the heating wires: material; structure; wire locations or winding axes location within the common heating coil; wire diameter, diameters of a turn of the first and second heating wires; sizes and structures of cross sectional areas, surface profiles, length, and others.

Further provided is an electronic smoking device with the inventive atomizer.

The characteristics, features and advantages of this invention and the manner in which they are obtained as described above, will become more apparent and be more clearly understood in connection with the following description of exemplary embodiments, which are explained with reference to the accompanying drawings.

### BRIEF DESCRIPTION OF THE DRAWINGS

In the drawings, same element numbers indicate same elements in each of the views:
- Figure 1: is a schematic cross-sectional illustration of an exemplary e-cigarette;
- Figure 2: is a schematic view of an atomizer in a first embodiment;
- Figure 3: is a section view through the atomizer of Figure 2;
- Figure 4: is a section view through an atomizer in a second embodiment;
- Figure 5: is a view of a heating coil perpendicular to its winding axis in a third embodiment, however, not forming part of the present invention;
- Figure 6: is a view of a heating coil perpendicular to its winding axis in a fourth embodiment;
- Figure 7: is a schematic view of an atomizer in a fifth embodiment;
- Figure 8: is a vertical cut through an atomizer in a sixth embodiment;
- Figure 9: shows a vertical cut through an atomizer in a seventh embodiment; and
- Figure 10: shows a cartomizer with an atomizer in an eighth embodiment.

### DESCRIPTION OF THE PREFERRED EMBODIMENTS

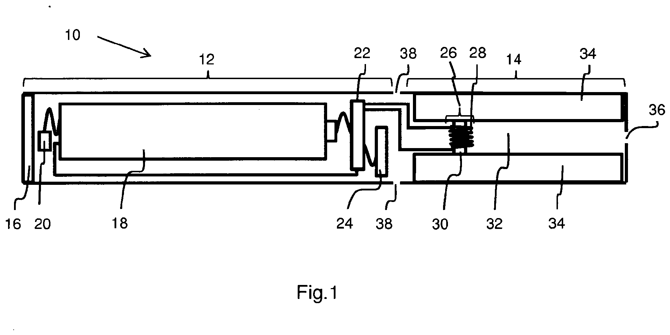

Throughout the following, an electronic smoking device will be exemplarily described with reference to an e-cigarette. As is shown in Figure 1, an e-cigarette 10 typically has a housing comprising a cylindrical hollow tube having an end cap 16. The cylindrical hollow tube may be single piece or a multiple piece tube. In Figure 1, the cylindrical hollow tube is shown as a two piece structure having a battery portion 12 and an atomizer/liquid reservoir portion 14. Together the battery portion 12 and the atomizer/liquid reservoir portion 14 form a cylindrical tube which is approximately the same size and shape as a conventional cigarette, typically about 100 mm with a 7.5 mm diameter, although lengths may range from 70 to 150 or 180 mm, and diameters from 5 to 20 mm.

The battery portion 12 and atomizer/liquid reservoir portion 14 are typically made of steel or hardwearing plastic and act together with the end cap 16 to provide a housing to contain the components of the e-cigarette 10. The battery portion 12 and an atomizer/liquid reservoir portion 14 may be configured to fit together by a friction push fit, a snap fit, or a bayonet attachment, magnetic fit, or screw threads. The end cap 16 is provided at the front end of the battery portion 12. The end cap 16 may be made from translucent plastic or other translucent material to allow an LED 20 positioned near the end cap to emit light through the end cap. The end cap can be made of metal or other materials that do not allow light to pass.

An air inlet may be provided in the end cap, at the edge of the inlet next to the cylindrical hollow tube, anywhere along the length of the cylindrical hollow tube, or at the connection of the battery portion 12 and the atomizer/liquid reservoir portion 14. Figure 1 shows a pair of air inlets 38 provided at the intersection between the battery portion 12 and the atomizer/liquid reservoir portion 14.

A battery 18, a light emitting diode (LED) 20, control electronics 22 and optionally an airflow sensor 24 are provided within the cylindrical hollow tube battery portion 12. The battery 18 is electrically connected to the control electronics 22, which are electrically connected to the LED 20 and the airflow sensor 24. In this example the LED 20 is at the front end of the battery portion 12, adjacent to the end cap 16 and the control electronics 22 and airflow sensor 24 are provided in the central cavity at the other end of the battery 18 adjacent the atomizer/liquid reservoir portion 14.

The airflow sensor 24 acts as a puff detector, detecting a user puffing or sucking on the atomizer/liquid reservoir portion 14 of the e-cigarette 10. The airflow sensor 24 can be any suitable sensor for detecting changes in airflow or air pressure such a microphone switch including a deformable membrane which is caused to move by variations in air pressure. Alternatively the sensor may be a Hall element or an electro-mechanical sensor.

The control electronics 22 are also connected to an atomizer 26. In the example shown, the atomizer 26 includes a heating coil 28 which is wrapped around a wick 30 extending across a central passage 32 of the atomizer/liquid reservoir portion 14. The coil 28 may be positioned anywhere in the atomizer 26 and may be transverse or parallel to the liquid reservoir 34. The wick 30 and heating coil 28 do not completely block the central passage 32. Rather an air gap is provided on either side of the heating coil 28 enabling air to flow past the heating coil 28 and the wick 30.

The central passage 32 is surrounded by a cylindrical liquid reservoir 34 with the ends of the wick 30 abutting or extending into the liquid reservoir 34. The wick 30 may be a porous material such as a bundle of fiberglass fibers, with liquid in the liquid reservoir 34 drawn by capillary action from the ends of the wick 30 towards the central portion of the wick 30 encircled by the heating coil 28.

The liquid reservoir 34 may alternatively include wadding soaked in liquid which encircles the central passage 32 with the ends of the wick 30 abutting the wadding. In other embodiments the liquid reservoir 34 may comprise a toroidal cavity arranged to be filled with liquid and with the ends of the wick 30 extending into the toroidal cavity.

An air inhalation port 36 is provided at the back end of the atomizer/liquid reservoir portion 14 remote from the end cap 16. The inhalation port 36 may be formed from the cylindrical hollow tube atomizer/liquid reservoir portion 14 or maybe formed in an end cap.

In use, a user sucks on the e-cigarette 10. This causes air to be drawn into the e-cigarette 10 via one or more air inlets, such as air inlets 38 and to be drawn through the central passage 32 towards the air inhalation port 36. The change in air pressure which arises is detected by the airflow sensor 24 which generates an electrical signal that is passed to the control electronics 22. In response to the signal, the control electronics 22 activate the heating coil 28 which causes liquid present in the wick 30 to be vaporized creating an aerosol (which may comprise gaseous and liquid components) within the central passage 32. As the user continues to suck on the e-cigarette 10, this aerosol is drawn through the central passage 32 and inhaled by the user. At the same time the control electronics 22 also activate the LED 20 causing the LED 20 to light up which is visible via the translucent end cap 16 mimicking the appearance of a glowing ember at the end of a conventional cigarette. As liquid present in the wick 30 is converted into an aerosol more liquid is drawn into the wick 30 from the liquid reservoir 34 by capillary action and thus is available to be converted into an aerosol through subsequent activation of the heating coil 28.

Some e-cigarette are intended to be disposable and the electric power in the battery 18 is intended to be sufficient to vaporize the liquid contained within the liquid reservoir 34 after which the e-cigarette 10 is thrown away. In other embodiments the battery 18 is rechargeable and the liquid reservoir 34 is refillable. In the cases where the liquid reservoir 34 is a toroidal cavity, this may be achieved by refilling the liquid reservoir 34 via a refill port. In other embodiments the atomizer/liquid reservoir portion 14 of the e-cigarette 10 is detachable from the battery portion 12 and a new atomizer/liquid reservoir portion 14 can be fitted with a new liquid reservoir 34 thereby replenishing the supply of liquid. In some cases, replacing the liquid reservoir 34 may involve replacement of the heating coil 28 and the wick 30 along with the replacement of the liquid reservoir 34. A replaceable unit comprising the atomizer 26 and the liquid reservoir 34 is called a cartomizer.

The new liquid reservoir 34 may be in the form of a cartridge having a central passage 32 through which a user inhales aerosol. In other embodiments, aerosol may flow around the exterior of the cartridge 32 to an air inhalation port 36.

Of course, in addition to the above description of the structure and function of a typical e-cigarette 10, variations also exist. For example, the LED 20 may be omitted. The airflow sensor 24 may be placed adjacent the end cap 16 rather than in the middle of the e-cigarette. The airflow sensor 24 may be replaced with a switch which enables a user to activate the e-cigarette manually rather than in response to the detection of a change in air flow or air pressure.

Different types of atomizers may be used. Thus for example, the atomizer may have a heating coil in a cavity in the interior of a porous body soaked in liquid. In this design aerosol is generated by evaporating the liquid within the porous body either by activation of the coil heating the porous body or alternatively by the heated air passing over or through the porous body. Alternatively the atomizer may use a piezoelectric atomizer to create an aerosol either in combination or in the absence of a heater.

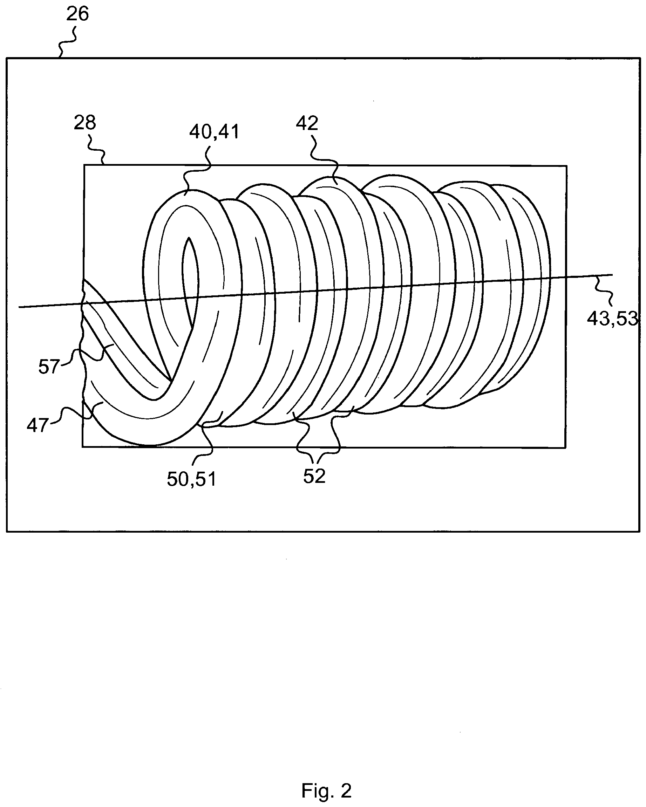

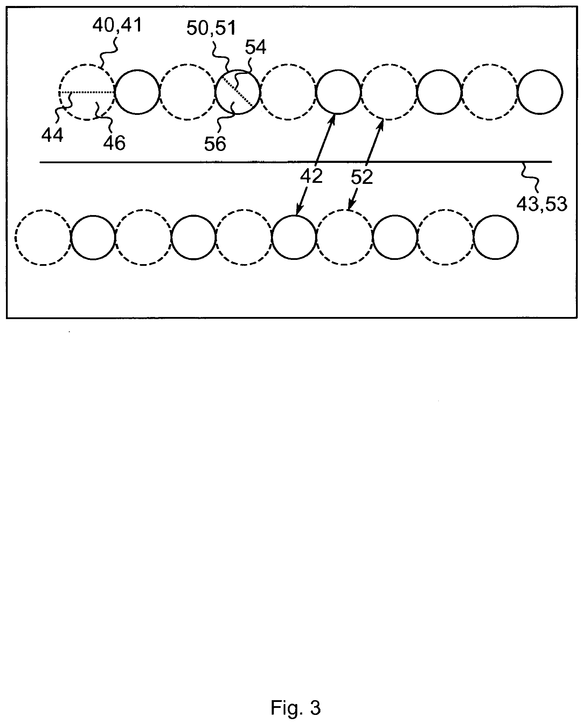

Figure 2 shows a schematic view of an atomizer 26 in a first embodiment. Figure 3 shows a cut through the inventive atomizer 26 of Figure 2 in a plane going through a center or winding axis 43, 53 of the first and second heating wires 40, 50. The atomizer 26 as shown in Figure 2 can be incorporated into the e-cigarette 10 of Figure 1 or other e-cigarettes which employ an atomizer 26. An atomizer 26 atomizes or vaporizes the liquid stored in the e-cigarette 10 of Figure 1. The atomizer 26 in the embodiment shown in Figure 2 includes a heating coil 28. The heating coil 28 comprises at least a first heating wire 40 and a second heating wire 50. The first and second heating wires 40, 50 are wound together to form the common heating coil 28. The first and second heating wires 40, 50 are parallel to each other. Each of the heating wires 40, 50 which constitute the common heating coil 28 are wound to form a first heating coil 41 and a second heating coil 51, respectively. Each loop of a heating wire 40, 50 is in the following named a turn 42, 52. In Figure 2, the first and second heating coils 41, 51 extend from left to right with a horizontal center or winding axis 43, 53. The first heating wire 40 has two end portions 47 (one on the left, shown in Figure 2; one on the right, not shown) which are used to electrically contact the first heating wire 40. Similarly, the second heating wire 50 has two end portions 57 which are used to electrically contact the second heating wire 50. The first and second heating wires 40, 50 are electrically contacted in parallel.

As can be seen from Figure 2, the first and second heating coils 41, 51 extend in the same direction and are staggered along the winding or center axis 43, 53 with respect to each other such that one turn 42 of the first heating wire 40 is neighbored by a turn 52 of the second heating wire 50. In the center region of the heating coil 28, the one turn 42 of the first heating wire 40 is neighbored by two turns 52 of the second heating wire 50. The two heating coils 41, 51 are wound together to form a single or common heating coil 28. In other words, the first and second heating coils 41, 51 are displaced along the center axis 43, 53 with an offset such that at least one turn 42 of the first heating coil 41 is placed between two turns 52 of the second heating coil 51, vice versa. The center axis 43 of the first heating coil 41 is identical to a center axis 53 of the second heating coil 51. A turn 42 of the first heating wire 40 directly contacts two turns 52 of the second heating wire 50. This allows the heating coil 28 to heat up quickly and to reduce a delay until an aerosol is produced from the liquid.

The first and second heating wires 40, 50, or the first and second heating coils 41, 51 according to all embodiments of the invention have at least one different physical parameter resulting in different thermal properties of the heating wires 40, 50 and heating coils 41, 51. In the embodiment shown in Figure 2 and Figure 3, the diameter 44 of the first heating wire 40 is greater than the diameter 54 of the second heating wire 50. Both heating wires 40, 50 are solid wires with constant cross section 46, 56 and planar outer surfaces. Due to the different physical parameters, here different wire thicknesses, the first and second heating wires 40, 50 heat differently. The first and second heating wires 40, 50 have different heating profiles or heat transfer characteristics and heat up and cool down at different speeds. Thin wires like the second heating wire 50 heat up and cool down very fast reducing the delay until an aerosol is produced by the atomizer 26 in contact with a liquid of an e-cigarette 10. Also, thin wires generally reach a maximum temperature faster while thick wires take longer to heat up but also retain their heat for longer.

The combination of at least two heating wires 40, 50 having different physical parameters provides in comparison with a heating coil consisting of a single heating wire a more complex heat transfer characteristic of an atomizer 26 in an e-cigarette 10 and thus a more complex aerosol generation upon contact with the liquid stored in the e-cigarette 10. The vaping experience may become more multidimensional using the different thermal properties of different heating wires 40, 50 combined into a common heating coil 28. In the state of the art, this has been achieved with staggered power delivery to a single wire, while according to an embodiment such electronics could be omitted or structured less complex resulting in a cheaper and simpler way to achieve a similar goal with improved performance outcomes of the atomizer 26 and consequently the e-cigarette 10.

By using two different heating wires 40, 50 with different thermal properties, e. g. by using two different heating wire thicknesses together in a single heating coil 28, the surface area of the heating coil 28 for liquid contact is greater in comparison with a heating coil 28 formed from a single heating wire. This improves an aerosol generation in an electronic smoking device 10 the inventive atomizer 26 is supplied to.

Figure 3 shows the different diameters 44, 54 of the first and second heating wires 40, 50 resulting in different sizes of their cross-sectional areas 46, 56. The first heating wire 40 has a larger diameter 44 and thus a larger cross-sectional area 46 than the second heating wire 50. The thicker first heating wire 40 has a lower resistance and provides more heat than the second heating wire 50. The turns 42 of the first heating wire 40 directly contact the neighboring turns 52 of the second heating wire 50 resulting in a dense heat transfer characteristic of the common heating coil 28.

The first and second heating wires 40, 50 of the first embodiment shown in Figures 2 and 3 differ in a single physical parameter, its wire thickness. However, the invention is not limited thereto. The heating wires 40, 50 can differ in a physical parameter, like the structure or size of cross-sections, surface profiles, materials etc. Some examples will be described in the following embodiments. The different embodiments can also be combined together such that the heating wires 40, 50 differ in two, three or a larger number of physical parameters leading to a complex heating profile of the common heating coil 28.

For instance, the heating wires 40, 50 of Figure 2 and Figure 3 may also be formed of a different material. One heating wire 40, 50 may be formed of a metal, one of a ceramic. Or both may be formed of different metals. One heating wire 40, 50 may be formed of a compound material or alloy, the other may be formed of a single material. The group of possible heating wire materials may comprise, for example, nickel, chromium, iron, aluminum, copper and alloys thereof as well as ceramics. Different heating wire materials will lead to different thermal properties and heat characteristic of the different heating coils 41, 51 resulting in a complex heat transfer pattern of the common heating coil 28 formed thereof.

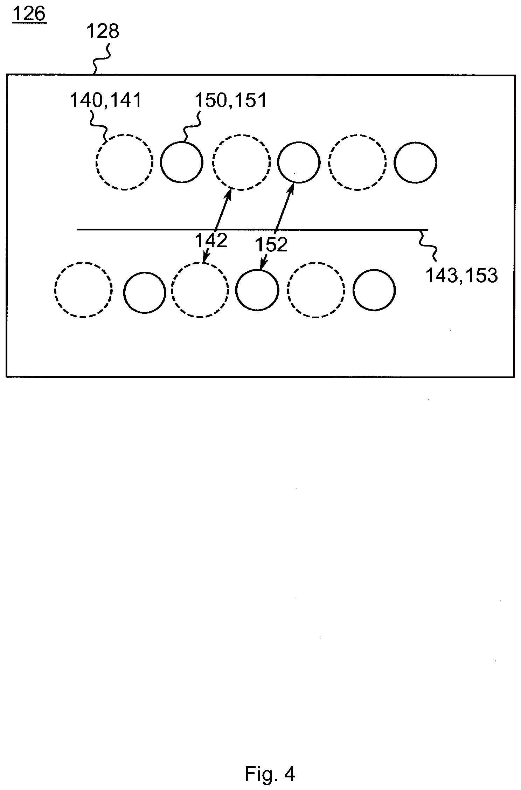

Figure 4 is a vertical cut through an atomizer 126 with a common heating coil 128 in a second embodiment. The second embodiment differs from the first embodiment of Figures 2 and 3 in that the turns 142, 152 of first and second heating coils 141, 151 do not directly contact each other but are spaced apart from each other along the center axis 143, 153 of the heating coils 141, 151. Again, in a region spaced apart from the outer turns 142 of the heating coil 128, one turn 142 of the first heating wire 140 is neighbored by two turns 152 of the second heating wire 150, vice versa. Yet, the turns 142 of the first heating wire 140 and the turns 152 of the second heating wire 150 do not directly contact each other but are spaced apart from each other. This can be achieved by forming the first and second heating coils 41, 51 rigid, e.g. by use of a ceramic or metal material. Spacing apart the two heating coils 141, 151 results in a larger wire surface usable for liquid contact, but also results in a less dense heating profile of the common heating coil 128.

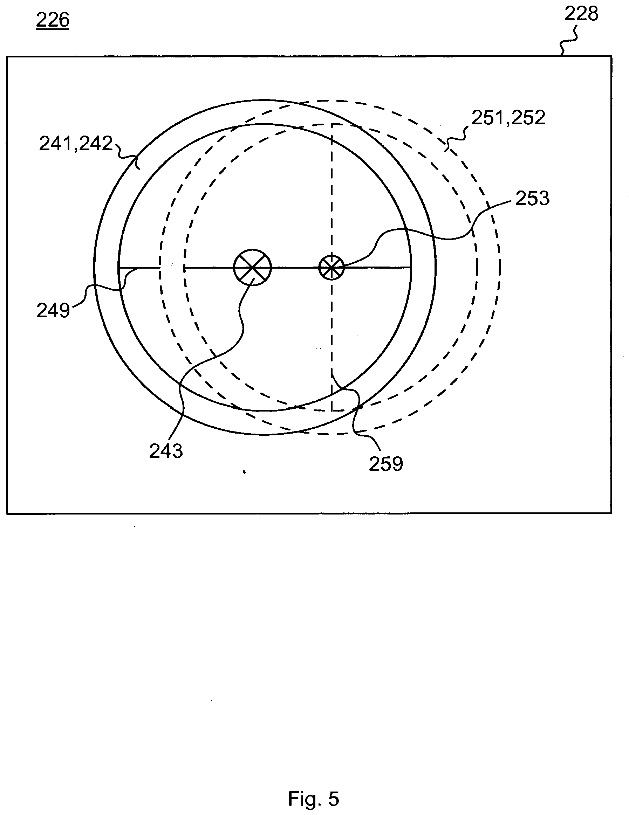

Figure 5 is a cut through an atomizer 226 with a common heating coil 228 perpendicular to its winding axis in a third embodiment, however not forming part of the present invention. Thus, the view is through the heating coil 228 along its length. Here, the first heating coil 241 and the second heating coil 251 have the same diameter 249, 259 of a turn 242, 252, but their center axes 243, 253 are displaced from each other. The displacement of the heating coils 241, 251 increases the complexity of the heating profiles of the common heating coil 228. However, the displacement of the heating coils 241, 251 with respect to each other alone does not result in a different thermal property of the first heating coil 241 with respect to the second heating coil 251. In order to have different thermal properties, they have to differ in an additional physical parameter. In the embodiment shown, the first and second heating coils 241, 251 are formed of different materials. A relative displacement of the second heating coil 251 may be limited such that the cross-sections of the turns 242, 252 of the first and second heating coils 241, 251 overlap. Preferably, the displacement of the center axis 253 of the second heating coil 251 from the center axis of the first heating coil 241 may be lower than a radius of a turn 42 of the first heating coil 241.

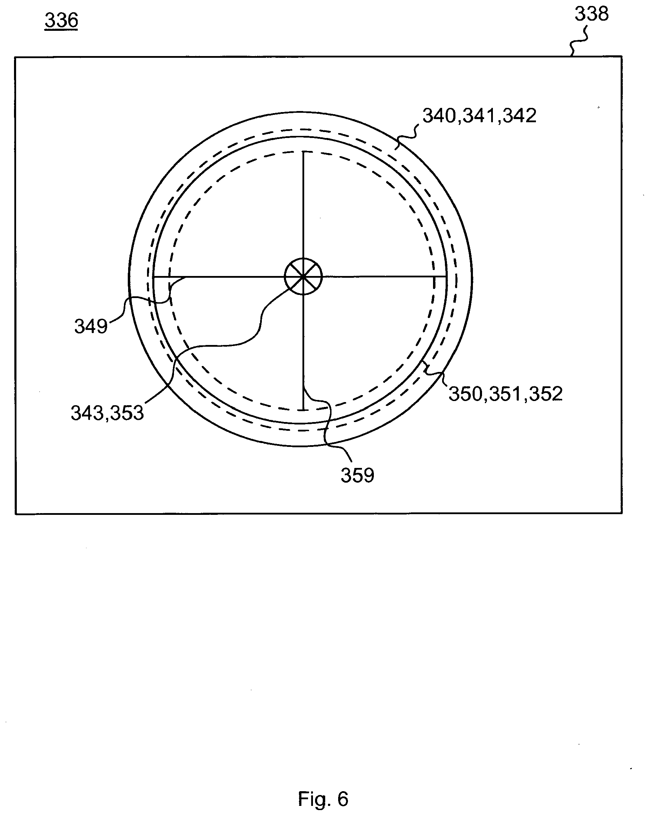

Figure 6 is a view of an atomizer perpendicular to its winding axis in a fourth embodiment. In comparison to the third embodiment in Figure 5, the heating coils 341, 351 have a common center axis 343, 353, but the diameter of a turn 342 of the first heating coil 341 is larger than the diameter of a turn 352 of the second heating coil 351. Thus, along the length of the common heating coil 328, its surface profile consists of valleys and peaks resulting in a complexity of a heat profile of the common heating coil 328. The two heating wires 340, 350 of this embodiment may have the same wire thicknesses but due to their different winding diameters 349, 359, the resulting heating coil 328 has a complex shape. In the preferred embodiment shown, the size of the winding diameter 359 of the second heating coil 351 is such that the cross-sections of the heating wires 340, 350 would still overlap in the cut shown in Figure 6.

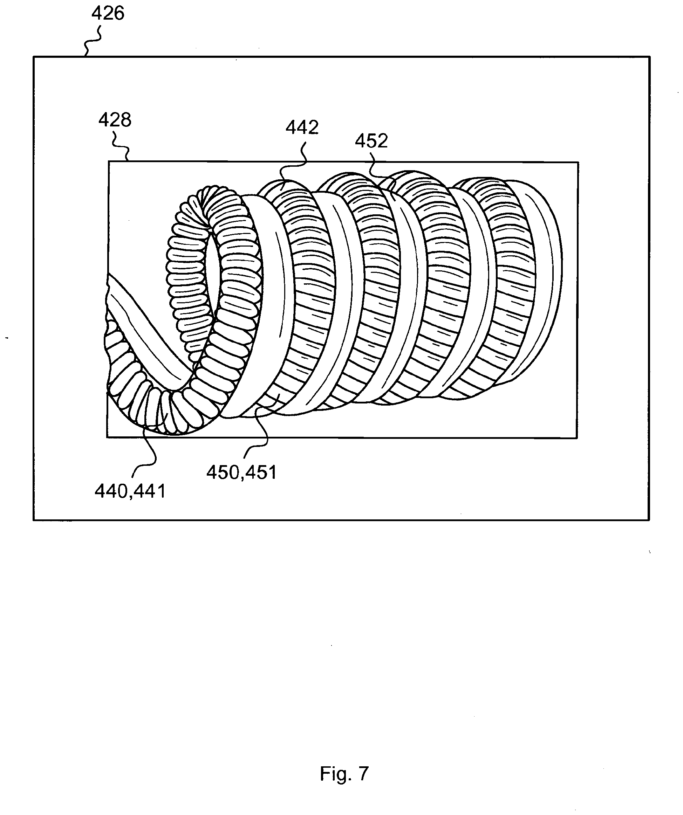

Figure 7 is a schematic view of an atomizer 426 in a fifth embodiment. The fifth embodiment differs from the first embodiment in Figure 2 in that the structure of the two heating wires 440, 450 differs. The first heating wire 440 is a twisted ribbon wire formed of a helix of spiral before it is wound to form the heating coil 428 together with second heating wire 450. Thus, the first heating wire 440 has a through-hole in its middle extending along its length and has varying cross sections 446 along its length. The second heating wire 450 is a solid wire with a non-varying circular cross-section 456 and a planar surface. Due to their different structure, the first and second heating wires 440, 450 shown in Figure 2 have different surface profiles. The surface profile of the first heating wire 440 before being wound into a heating coil 441 consists of alternating valleys and peaks, whereas the surface profile of the second heating wire 450 is planar. The valleys of the first heating wire 440 increase the wire area usable for liquid contact and therefore improve the aerosol formation, while the solid second heating wire 450 provides more heat since it has a lower resistance than the first heating wire 440.

The examples of a ribbon heating wire 440 and a round heating wire 450 are not limiting. Any physical shape or structure of the heating wires 440, 450 can be envisaged, e.g. a stranded wire, an oval wire, a wire with a surface structure etc.

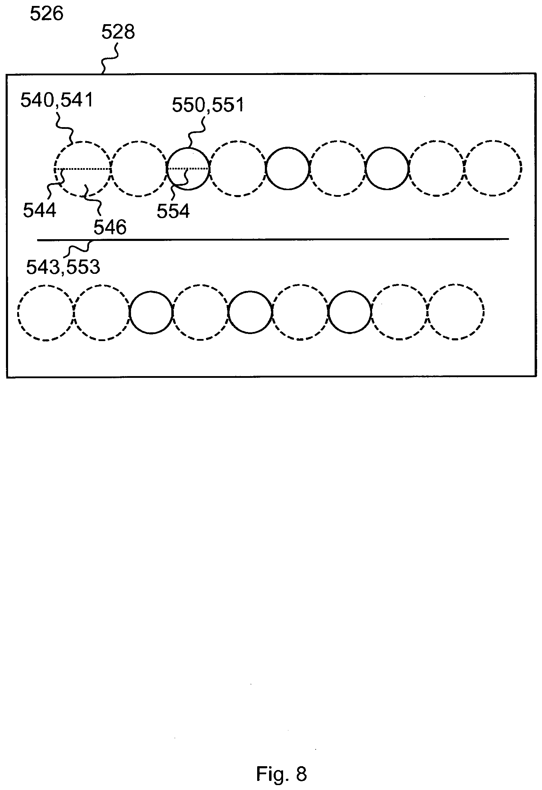

Figure 8 is a vertical cut through an atomizer 526 in a sixth embodiment. Here, the first and second heating wires 540, 550 have a different length and thus contribute with a different number of turns 542, 552 to the common heating coil 528. As exemplarily shown, while the first heating wire 540 contributes with six turns 542 to the common heating coil 528, the second heating wire 550 is shorter and contributes only with three turns 552 to the common heating coil 528.

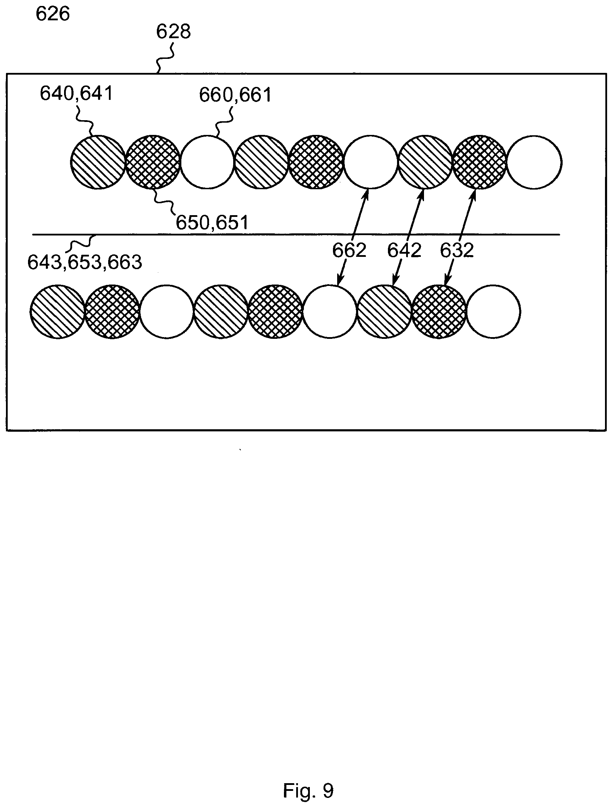

Figure 9 shows a vertical cut through an atomizer 626 in a seventh embodiment. In this embodiment, a third heating wire 660 is additionally provided, wherein the first, second and third heating wires 640, 650, 660 are wound together to form the heating coil 628. One turn 642 of the first heating wire 640 contacts on one side a turn 652 of a second heating wire 650 and on the other side one turn 662 of the third heating wire 660. The first, second and third heating wires 640, 650, 660 in Figure 9 are formed of different materials. While the first heating wire 640 is formed of a nickel-chromium alloy, the second heating wire 650 is formed of iron-chromium-aluminum alloy and the third heating wire 660 is formed of a nickel-iron alloy. Due to their different materials, the heating wires 640, 650, 660 have a different resistivity and therefore heat differently. Apart from their materials, the heating wires 640, 650, 660 do not differ in the embodiment shown. However, the third heating wire 660 could differ from the first and second heating wires 640, 650, respectively, also in any other physical parameter apart of its material.

Also more than three heating wires could be provided to form the common heating coil of the atomizer. Preferably, at least two of the group of heating wires differ in at least one physical parameter. In another embodiment, none of the provided heating wires would have an identical set of physical parameters as any other of the group of heating wires in the common heating coil.

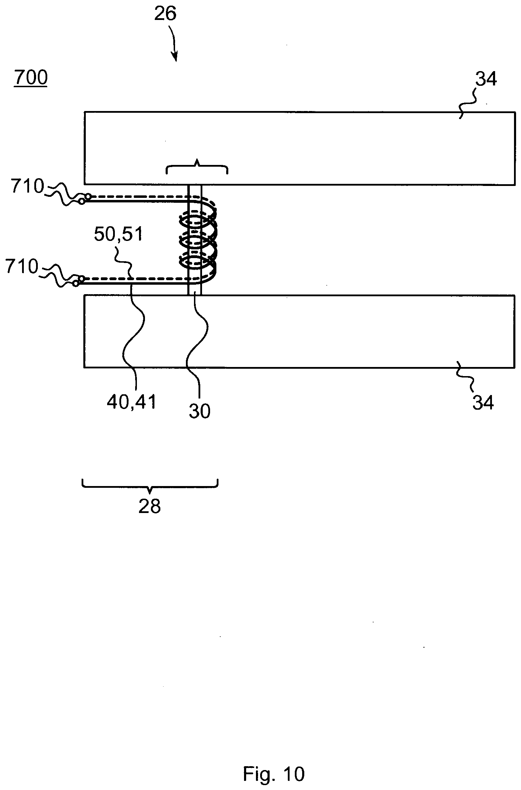

Electronic smoking devices may be structured such that the liquid reservoir can be removed from an electronic cigarette together with the atomizer and can be replaced by a new, refilled atomizer/liquid reservoir portion 14 being called a cartomizer. Fig. 10 shows a cartomizer 700 for an electronic smoking device 10 according to an embodiment. The cartomizer 700 comprises a liquid reservoir 34 as described in the context of Fig. 1 with an atomizer 26 of Fig. 2. However, all other embodiments of the atomizer described in the context of this invention may be used as an atomizer in Fig. 10. The atomizer/liquid reservoir portion 14 can be separated from the battery portion 12 and the cartomizer 700 can be removed and replaced. The first and second heating wires 40, 50, respectively the first and second heating coils 41, 51 of the atomizer 26 have electronic contacts 710, which upon fixation of the atomizer/liquid reservoir portion 14 to the battery portion 12 provide electrical contact to the battery 18. However, the electronic smoking device may be configured differently, for instance with an opening in its housing through which the cartomizer is removable or replaceable.

In summary, an atomizer for an electronic smoking device is provided comprising at least a first heating wire and a second heating wire. The first and second heating wires differ in at least one physical parameter and are wound together to form a common heating coil. Due to the at least one different physical parameter, the first and second heating wires have different thermal properties and heat differently. The first and second heating wires have different heating profiles or heat transfer characteristics. The first and second heating wires heat up and cool down at different speeds. In one aspect, a greater surface area for liquid contact may be provided. Different physical parameters of the first and second heating wires may relate to at least one of the following features:
- the first and second heating wires are formed of a different material,
- the first and second heating wires have a different structure,
- the locations of the first and second heating wires within the common heating coil 28 differ,
- the diameter of the first and second heating wires differ,
- the diameters of a turn of the first and second heating wires differ,
- the first and second heating wires have different sizes or structures of cross sectional areas in a common cut through the heating coil,
- one of the first and second heating wires has a varying cross section while the other has a differently varying cross section or a constant cross section,
- the first and second heating wires have different surface profiles, surface treatments, or surface coatings, and
- the first and second heating wires have a different length.

Only one, a group of or all of the above features may be present in an embodiment of the invention.

Preferably, the first heating wire is wound into a first heating coil, the second heating wire is wound into a second heating coil, wherein the first and second coils extend in the same direction and are staggered along the winding axis with respect to each other such that one turn of the first heating wire is neighbored by at least one turn, preferably two turns of the second heating wire. The two heating coils are wound together to form a single heating coil. Preferably, the turns of the two heating coils are displaced along the winding axis with an offset such that one turn of the first heating coil is placed between two turns of the second heating coil, vice versa. Preferably, the center axis of the first heating coil is parallel to a center axis of the second heating coil. In one aspect, the center axis of the first heating coil is identical to a center axis of the second heating coil. This saves space and increases the heating performance of the heating coil.

In one aspect, a turn of the first heating wire directly contacts two turns of the second heating wire. This would allow the heating coil to heat up quickly and to reduce the delay until aerosol is produced. In one aspect, a diameter of the first heating wire is different from the diameter of the second heating wire. This may provide a simple way to achieve different heating profiles. In one aspect, the first heating wire and the second heating wire are made of different materials. In another aspect, a surface profile of the first heating wire is different from a surface profile of the second heating wire. Preferably, a cross section of a first heating wire varies differently along its length compared to a cross section of the second heating wire. A cross section of a first heating wire may vary along its length wherein the cross section of the second heating wire may be constant. In a vertical cut through the heating coil, a cross section of a first heating wire 40 may be different from a cross section of the second heating wire. For example, although wires having a round cross section are commonly available in various materials and diameters, one or more of the heating wires, or parts of it, may have a non-round cross section, such as a flatter ribbon-like wire.

The number of end portions of the heating coil is double the number of heating wires. Preferably, the first end portion of the first heating wire and the end portion of the second heating wire are coupled together into a first common contact portion and the second end portions of the first and second heating wires are coupled together into a second common contact portion, respectively.

In yet another embodiment, a third heating wire is additionally provided, wherein the first, second and third heating wires are wound together to form the heating coil. Preferably, one turn of the first heating wires contacts on one side one turn of a second heating wire and on the other side one turn of the third heating wire. The third heating wire differs from the first and/or second heating wire in at least one physical parameter, e.g. its material; its cross section; its variation of its cross section along its length; its length; and its surface profile such that the third heating wire has a different thermal property than the first and second heating wires.

In one embodiment, an electronic smoking device is provided comprising: a housing, a liquid reservoir provided inside the housing, and an atomizer as described above. The invention is not limited to a heating wire with a wick but may be used with any other element for providing the liquid to the heating wire.

While this invention has been described in connection with what is presently considered to be practical exemplary embodiments, it is to be understood that the invention is not limited to the disclosed embodiments, but, on the contrary, is intended to cover various modifications and equivalent arrangements included within the scope of the appended claims.

### LIST OF REFERENCE SIGNS

- 10: e-cigarette
- 12: battery portion
- 14: atomizer/liquid reservoir portion
- 16: end cap
- 18: battery
- 20: light emitting diode (LED)
- 22: control electronics
- 24: airflow sensor
- 26: atomizer
- 28: heating coil
- 30: wick
- 32: central passage
- 34: liquid reservoir
- 36: air inhalation port
- 38: air inlets
- 40: first heating wire
- 41: first heating coil
- 42: turn of first heating wire
- 43: center axis of first heating wire
- 44: diameter of first heating wire
- 45: surface profile of first heating wire
- 46: cross section of first heating wire
- 47: first end portion of first heating wire
- 48: second end portion of first heating wire
- 49: diameter of turn of a first heating wire
- 50: second heating wire
- 51: second heating coil
- 52: turn of second heating wire
- 53: center axis of second heating wire
- 54: diameter of second heating wire
- 55: surface profile of second heating wire
- 56: cross section of second heating wire
- 57: first end portion of second heating wire
- 58: second end portion of second heating wire
- 59: diameter of turn of a second heating wire

- 660: third heating coil
- 662: turn of third heating wire

- 700: cartomizer
- 710: electrical contacts of heating wires

## Claims

1. An atomizer (26) for an electronic smoking device (10) comprising at least a first heating wire (40) and a second heating wire (50), wherein the first and second heating wires (40, 50) are wound together to form a common heating coil (28), and wherein the first and second heating wires (40, 50) differ in at least one physical parameter resulting in different thermal properties of the first and second heating wires (40, 50),
**characterized in that**
the diameter (349) of a turn (342) of the first heating wire (340) differs from a diameter (359) of a turn (352) of the second heating wire (350), and
the inner winding diameter (349) of the first heating wire (340) differs from the inner winding diameter (359) of the second heating wire (350).

2. The atomizer according to claim 1, wherein the first heating wire (40) is wound into a first heating coil (41), the second heating wire (50) is wound into a second heating coil (51), wherein the first and second coils (41, 51) extend in the same direction and are staggered with respect to each other such that one turn (42) of the first heating wire (40) is neighbored by at least one turn (52) of the second heating wire (50).

3. The atomizer according to claim 2, wherein a center axis (43) of the first heating coil (41) is parallel to a center axis (53) of the second heating coil (51).

4. The atomizer according to claim 2, wherein a center axis (43) of the first heating coil (41) is identical to a center axis (53) of the second heating coil (51).

5. The atomizer according to any of the previous claims, wherein a turn (42) of the first heating wire (40) directly contacts two turns (52) of the second heating wire (50).

6. The atomizer according to any of the previous claims, wherein a diameter (44) of the first heating wire (40) is different from the diameter (54) of the second heating wire (50).

7. The atomizer according to any of the previous claims, wherein the first heating wire (40) and the second heating wire (50) are made of different materials.

8. The atomizer according to any of the previous claims, wherein a surface profile (445) of the first heating wire (440) is different from a surface profile (455) of the second heating wire (450).

9. The atomizer according to any of the previous claims, wherein a cross section (46) of a first heating wire (40) varies differently along its length than a cross section (56) of the second heating wire (50).

10. The atomizer according to any of the previous claims 1 to 8, wherein a cross section of a first heating wire (440) varies along its length wherein the cross section of the second heating wire (450) is constant.

11. The atomizer according to any of the previous claims, wherein in a vertical cut through the heating coil (28), a cross section (46) of a first heating wire (40) is different from a cross section (56) of the second heating wire (42).

12. The atomizer according to any of the previous claims, wherein the length of the first heating wire (540) differs from the length of the second heating wire (550).

13. The atomizer according to any of the previous claims, wherein a third heating wire (660) is additionally provided, wherein the first, second and third heating wires (640, 650, 660) are wound together to form the heating coil (628), and wherein the third heating wire (660) differs from each the first and the second heating wires (640, 650) in at least one physical parameter resulting in different thermal properties of the first and second heating wires (640, 650).

14. A cartomizer (700) for an electronic smoking device (10) comprising:
- a liquid reservoir (34), and
- an atomizer (26) according to one of the previous claims configured to atomize a liquid stored in the liquid reservoir (34).

15. An electronic smoking device (10) comprising:
- a housing (12, 14);
- a liquid reservoir (34) provided inside the housing (12, 14), and
- an atomizer (26) according to one of the previous claims 1 to 13 provided to atomize a liquid stored in the liquid reservoir (34).

## Patentansprüche

1. Zerstäuber (26) für eine elektronische Rauchvorrichtung (10), umfassend mindestens einen ersten Heizdraht (40) und einen zweiten Heizdraht (50), wobei der erste und der zweite Heizdraht (40, 50) zusammengewickelt sind, um eine gemeinsame Heizspule (28) auszubilden, und wobei der erste und der zweite Heizdraht (40, 50) sich hinsichtlich mindestens eines physischen Parameters unterscheiden, um verschiedene Wärmeeigenschaften des ersten und des zweiten Heizdrahtes (40, 50) zu ergeben,
**dadurch gekennzeichnet, dass**
der Durchmesser (349) einer Windung (342) des ersten Heizdrahtes (340) sich von einem Durchmesser (359) einer Windung (352) des zweiten Heizdrahtes (350) unterscheidet und der innere Wicklungsdurchmesser (349) des ersten Heizdrahtes (340) sich von dem inneren Wicklungsdurchmesser (359) des zweiten Heizdrahtes (350) unterscheidet.

2. Zerstäuber nach Anspruch 1, wobei der erste Heizdraht (40) zu einer ersten Heizspule (41) aufgewickelt ist und der zweite Heizdraht (50) zu einer zweiten Heizspule (51) aufgewickelt ist,
wobei die erste und die zweite Spule (41, 51) sich in dieselbe Richtung erstrecken und zueinander derart versetzt sind, dass eine Windung (42) des ersten Heizdrahtes (40) zu mindestens einer Windung (52) des zweiten Heizdrahtes (50) benachbart ist.

3. Zerstäuber nach Anspruch 2, wobei eine Mittelachse (43) der ersten Heizspule (41) parallel zu einer Mittelachse (53) der zweiten Heizspule (51) verläuft.

4. Zerstäuber nach Anspruch 2, wobei eine Mittelachse (43) der ersten Heizspule (41) identisch mit einer Mittelachse (53) der zweiten Heizspule (51) ist.

5. Zerstäuber nach einem der vorangehenden Ansprüche, wobei eine Windung (42) des ersten Heizdrahtes (40) zwei Windungen (52) des zweiten Heizdrahtes (50) direkt berührt.

6. Zerstäuber nach einem der vorangehenden Ansprüche, wobei ein Durchmesser (44) des ersten Heizdrahtes (40) sich von dem Durchmesser (54) des zweiten Heizdrahtes (50) unterscheidet.

7. Zerstäuber nach einem der vorangehenden Ansprüche, wobei der erste Heizdraht (40) und der zweite Heizdraht (50) aus verschiedenen Materialien bestehen.

8. Zerstäuber nach einem der vorangehenden Ansprüche, wobei ein Oberflächenprofil (445) des ersten Heizdrahtes (440) sich von einem Oberflächenprofil (455) des zweiten Heizdrahtes (450) unterscheidet.

9. Zerstäuber nach einem der vorangehenden Ansprüche, wobei ein Querschnitt (46) des ersten Heizdrahtes (40) entlang seiner Länge anders variiert als ein Querschnitt (56) des zweiten Heizdrahtes (50).

10. Zerstäuber nach einem der Ansprüche 1 bis 8, wobei ein Querschnitt eines ersten Heizdrahtes (440) entlang seiner Länge variiert, wobei der Querschnitt des zweiten Heizdrahtes (450) konstant ist.

11. Zerstäuber nach einem der vorangehenden Ansprüche, wobei, in einem vertikalen Schnitt durch die Heizspule (28), ein Querschnitt (46) eines ersten Heizdrahtes (40) sich von einem Querschnitt (56) des zweiten Heizdrahtes (42) unterscheidet.

12. Zerstäuber nach einem der vorangehenden Ansprüche, wobei die Länge des ersten Heizdrahtes (540) sich von der Länge des zweiten Heizdrahtes (550) unterscheidet.

13. Zerstäuber nach einem der vorangehenden Ansprüche, wobei ein dritter Heizdraht (660) zusätzlich vorgesehen ist, wobei der erste, der zweite und der dritte Heizdraht (640, 650, 660) zusammengewickelt sind, um die Heizspule (628) auszubilden, und wobei der dritte Heizdraht (660) sich von jedem aus dem ersten und dem zweiten Heizdraht (640, 650) hinsichtlich mindestens eines physischen Parameters unterscheidet, um verschiedene Wärmeeigenschaften des ersten und des zweiten Heizdrahtes (640, 650) zu ergeben.

14. Cartomizer (700) für eine elektronische Rauchvorrichtung (10), umfassend:
- ein Flüssigkeitsreservoir (34) und
- einen Zerstäuber (26) nach einem der vorangehenden Ansprüche, der dazu ausgestaltet ist, eine in dem Flüssigkeitsreservoir (34) gespeicherte Flüssigkeit zu zerstäuben.

15. Elektronische Rauchvorrichtung (10), umfassend:
- ein Gehäuse (12, 14);
- ein Flüssigkeitsreservoir (34), das innerhalb des Gehäuses (12, 14) vorgesehen ist, und
- einen Zerstäuber (26) nach einem der Ansprüche 1 bis 13, der dazu vorgesehen ist, eine in dem Flüssigkeitsreservoir (34) gespeicherte Flüssigkeit zu zerstäuben.

## Revendications

1. Atomiseur (26) pour dispositif de cigarette électronique (10) comprenant au moins un premier fil chauffant (40) et un deuxième fil chauffant (50), les premier et deuxième fils chauffants (40, 50) étant enroulés ensemble pour former un serpentin chauffant (28) commun, et les premier et deuxième fils chauffants (40, 50) différant par au moins un paramètre physique entraînant différentes propriétés thermiques des premier et deuxième fils chauffants (40, 50),
**caractérisé en ce que**
le diamètre (349) d'une spire (342) du premier fil chauffant (340) diffère d'un diamètre (359) d'une spire (352) du deuxième fil chauffant (350), et
le diamètre d'enroulement intérieur (349) du premier fil chauffant (340) diffère du diamètre d'enroulement intérieur (359) du deuxième fil chauffant (350).

2. Atomiseur selon la revendication 1, dans lequel le premier fil chauffant (40) est enroulé en un premier serpentin chauffant (41), le deuxième fil chauffant (50) est enroulé en un deuxième serpentin chauffant (51),
les premier et deuxième serpentins (41, 51) s'étendant dans la même direction et étant décalés l'un par rapport à l'autre de telle sorte qu'une spire (42) du premier fil chauffant (40) est avoisinée par au moins une spire (52) du deuxième fil chauffant (50).

3. Atomiseur selon la revendication 2, dans lequel un axe central (43) du premier serpentin chauffant (41) est parallèle à un axe central (53) du deuxième serpentin chauffant (51).

4. Atomiseur selon la revendication 2, dans lequel un axe central (43) du premier serpentin chauffant (41) est identique à un axe central (53) du deuxième serpentin chauffant (51).

5. Atomiseur selon l'une quelconque des revendications précédentes, dans lequel une spire (42) du premier fil chauffant (40) est en contact direct avec deux spires (52) du deuxième fil chauffant (50).

6. Atomiseur selon l'une quelconque des revendications précédentes, dans lequel un diamètre (44) du premier fil chauffant (40) est différent du diamètre (54) du deuxième fil chauffant (50).

7. Atomiseur selon l'une quelconque des revendications précédentes, dans lequel le premier fil chauffant (40) et le deuxième fil chauffant (50) sont fabriqués en matériaux différents.

8. Atomiseur selon l'une quelconque des revendications précédentes, dans lequel un profil de surface (445) du premier fil chauffant (440) est différent d'un profil de surface (455) du deuxième fil chauffant (450).

9. Atomiseur selon l'une quelconque des revendications précédentes, dans lequel une section transversale (46) d'un premier fil chauffant (40) varie différemment sur sa longueur qu'une section transversale (56) du deuxième fil chauffant (50).

10. Atomiseur selon une des revendications 1 à 8, dans lequel une section transversale d'un premier fil chauffant (440) varie sur sa longueur, dans lequel une section transversale du deuxième fil chauffant (450) est constante.

11. Atomiseur selon l'une quelconque des revendications précédentes, dans lequel, dans une coupe verticale à travers le serpentin chauffant (28), une section transversale (46) d'un premier fil chauffant (40) est différente d'une section transversale (56) du deuxième fil chauffant (42).

12. Atomiseur selon l'une quelconque des revendications précédentes, dans lequel la longueur du premier fil chauffant (540) diffère de la longueur du deuxième fil chauffant (550).

13. Atomiseur selon l'une quelconque des revendications précédentes, dans lequel un troisième fil chauffant (660) est prévu en plus, les premier, deuxième et troisième fils chauffants (640, 650, 660) étant enroulés ensemble pour former le serpentin chauffant (628), et le troisième fil chauffant (660) différant de chacun des premier et deuxième fils chauffants (640, 650) par au moins un paramètre physique entraînant des propriétés thermiques différentes des premier et deuxième fils chauffants (640, 650).

14. Atomiseur (700) pour dispositif de cigarette électronique (10), comprenant :
- un réservoir de liquide (34) et
- un atomiseur (26) selon une des revendications précédentes, conçu pour atomiser un liquide stocké dans le réservoir de liquide (34).

15. Dispositif de cigarette électronique (10) comprenant :
- un boîtier (12, 14) ;
- un réservoir de liquide (34) fourni dans le boîtier (12, 14) et
- un atomiseur (26) selon une des revendications 1 à 13, prévu pour atomiser un liquide stocké dans le réservoir de liquide (34).
